**Europäisches Patentamt**

**European Patent Office**

**Office européen des brevets**

(19)

(11) Veröffentlichungsnummer : **0 076 499**
**B1**

(12) **EUROPÄISCHE PATENTSCHRIFT**

(45) Veröffentlichungstag der Patentschrift :
02.01.86

(21) Anmeldenummer : 82109115.4

(22) Anmeldetag : 02.10.82

(51) Int. Cl.⁴ : **G 02 C  1/08**, G 02 C  5/14

(54) **Brille, insbesondere Schutzbrille.**

(30) Priorität : **05.10.81 DE 3139555**

(43) Veröffentlichungstag der Anmeldung :
**13.04.83 Patentblatt 83/15**

(45) Bekanntmachung des Hinweises auf die Patenterteilung : **02.01.86 Patentblatt 86/01**

(84) Benannte Vertragsstaaten :
**FR GB IT**

(56) Entgegenhaltungen :
**DE-A- 1 925 231**
**DE-B- 1 285 767**
**DE-B- 2 003 450**
**FR-A- 1 499 503**

(73) Patentinhaber : **Fondermann GmbH.**
**1, Diekerstrasse**
**D-5657 Haan (DE)**

(72) Erfinder : **Hoebink, Paul**
**25, Hacketäuer Strasse**
**D-5650 Solingen (DE)**
Erfinder : **Laibach, Friedrich**
**48, Böttinger Strasse**
**D-5657 Haan (DE)**

(74) Vertreter : **Tackenberg, Karl, Dipl.-Ing.**
**Birkenweiher 15**
**D-5650 Solingen (DE)**

## Beschreibung

Die Erfindung betrifft eine Brille, insbesondere eine Schutzbrille, mit geschlitzten Brillenglasfassungen und veränderbarer Inklination der Ohrenbügel, bei der ein jeder Ohrenbügel mit einer mit ihm gelenkig verbundenen Hülse auf einem an dem Brillenrahmen seitlich fest angeordneten, in Längsrichtung geteilten Zapfen gelagert ist, dessen Teilungsebene in die Schlitzebene der Brillenglasfassung übergeht, und bei der der Ohrenbügel in der zum Brillenrahmen eingestellten geneigten Lage mittels einer aus ineinandergreifenden Riffelungen bestehenden Rastsperre fixierbar ist, wobei die einen Riffelungen am inneren Umfang der Hülse des Ohrenbügels angeordnet sind.

Brillen, bei denen die Inklination der Ohrenbügel nach erfolgtem Einstellen mittels einer aus ineinandergreifenden Riffelungen bestehenden Rastsperre fixierbar ist, sind bekannt. Bei einer bekannten Brille, bei der der Ohrenbügel mit der Hülse um die Achse des an dem Brillenrahmen fest angeordneten Zapfens verschwenkbar ist, sind diejenigen Riffelungen der Rastsperre, die mit den am inneren Umfang der Hülse des Ohrenbügels angeordneten Riffelungen in Wirkverbindung stehen, am Umfang des Zapfens angeordnet. Diese Anordnung der Riffelungen der Rastsperre hat jedoch einen wesentlichen Nachteil, der in folgendem zu erblicken ist. Bei jedesmaligem Auswechseln eines Brillenglases muß die Brillenglasfassung an dem Schlitz geöffnet werden, zu welchem Zweck zuvor der Ohrenbügel von dem Zapfen abgezogen werden muß. Es läßt sich dabei und beim erneuten Aufschieben des Ohrenbügels auf den Zapfen nicht ausschließen, daß die Riffelungen, insbesondere Feinriffelungen, beschädigt werden.

Der Erfindung liegt die Aufgabe zugrunde, bei einer Brille der eingangs beschriebenen Gattung die Rastsperre so anzuordnen, daß bei Lösen des Ohrenbügels von dem Zapfen des Brillenrahmens und erneutem Aufschieben des Ohrenbügels auf den Zapfen eine Beschädigung der Riffelungen der Rastsperre mit Sicherheit vermieden ist.

Die Lösung der Aufgabe besteht erfindungsgemäß darin, daß

a) in die Hülse des Ohrenbügels eine Buchse eingesetzt ist, bei der die mit den am inneren Umfang der Hülse angeordneten Riffelungen der Rastsperre in Wirkverbindung stehenden Riffelungen außen am Mantel angeordnet sind,

b) die Buchse gemeinsam mit der Hülse auf den an dem Brillenrahmen angeordneten Zapfen drehfest aufgeschoben ist.

Der Zapfen ist vorzugsweise vierkantig und die Buchse mit einem entsprechenden Innenvierkant versehen.

Der Ohrenbügel ist nunmehr mit seiner Hülse um die Buchse verschwenkbar. Er wird zum Auswechseln eines Brillenglases mit der Buchse von dem Zapfen abgezogen und nach Auswechseln des Brillenglases mit der Buchse wieder auf den Zapfen aufgeschoben. Die Riffelungen der Rastsperre bleiben dabei im Eingriff und unterliegen dadurch keiner Beschädigung.

Eine vorteilhafte Weiterbildung der erfindungsgemäßen Brille besteht darin, daß die Riffelungen der Rastsperre in Gruppen so aufgeteilt sind, daß zwischen den einzelnen Gruppen Freiräume vorhanden sind.

Die Buchse wird bei der Montage in die Hülse des Ohrenbügels so eingesetzt, daß sie mit ihren Riffelungen in die Freiräume zwischen den zu Gruppen zusammengefaßten Riffelungen der Hülse eingreifen. Die Riffelungen werden dadurch nicht zwangsweise, sondern erst durch Verschwenken des Ohrenbügels miteinander in Eingriff gebracht, so daß auch bereits bei der Montage der Buchse eine Beschädigung der Riffelungen verhütet ist.

Eine weitere vorteilhafte Weiterbildung der erfindungsgemäßen Brille besteht darin, daß

a) die Buchse an ihrem einen Ende geschlossen ist und einen kreisrunden Vorsprung besitzt,

b) die Buchse an ihrem anderen Ende nach auswärts gerichtete, sich gegenüberliegende nasenartige Vorsprünge besitzt,

c) die Buchse mit ihren Vorsprüngen in Randausnehmungen der Hülse liegt,

d) der Mantel der Buchse in das offene Ende der Buchse auslaufende Schlitze aufweist.

Da die Buchse mit ihrem offenen Ende der Brillenglasfassung bis auf einen Spalt angenähert ist, die Buchse aber an ihrem anderen Ende geschlossen ist, kann in den Raum zwischen der Buchse und der Hülse kein Schmutz o. dgl. eindringen, so daß die Rastsperre weitgehend gegen Einflüsse von außen geschützt ist. Die Buchse ist ferner vor unbeabsichtigtem Lösen aus der Hülse gesichert.

Die Erfindung ist nachstehend in einem Ausführungsbeispiel anhand der Zeichnung näher erläutert. Es zeigt:

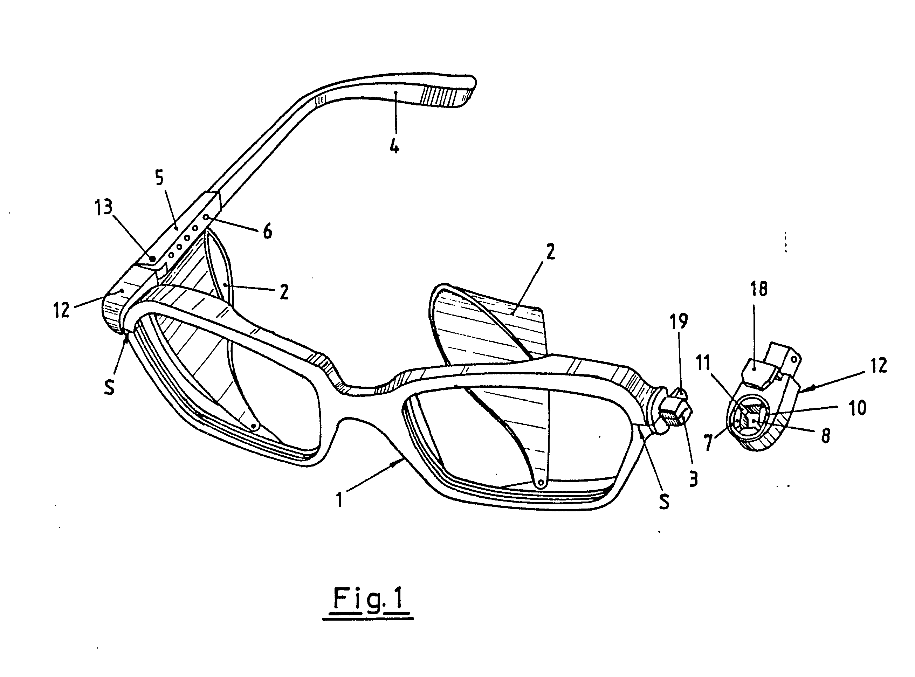

Figur 1 die Brille in perspektivischer Darstellung,

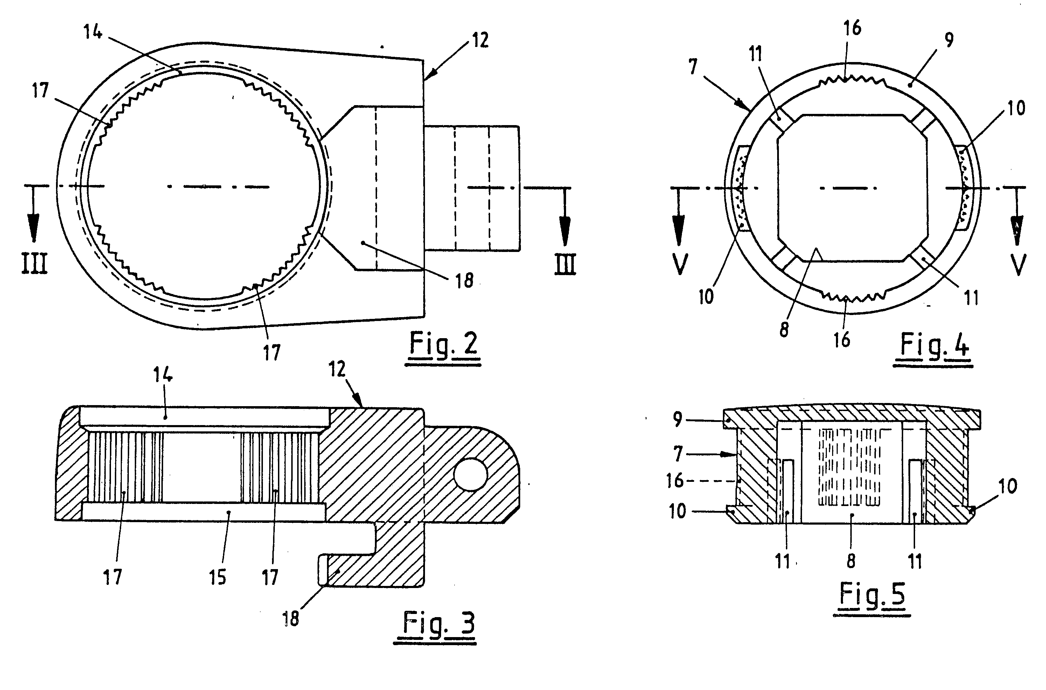

Figur 2 die Hülse in vergrößertem Maßstab in Draufsicht,

Figur 3 einen Schnitt nach der Linie III-III der Figur 2,

Figur 4 die Buchse in vergrößertem Maßstab in Draufsicht auf das offene Ende,

Figur 5 einen Schnitt nach der Linie V-V der Figur 4,

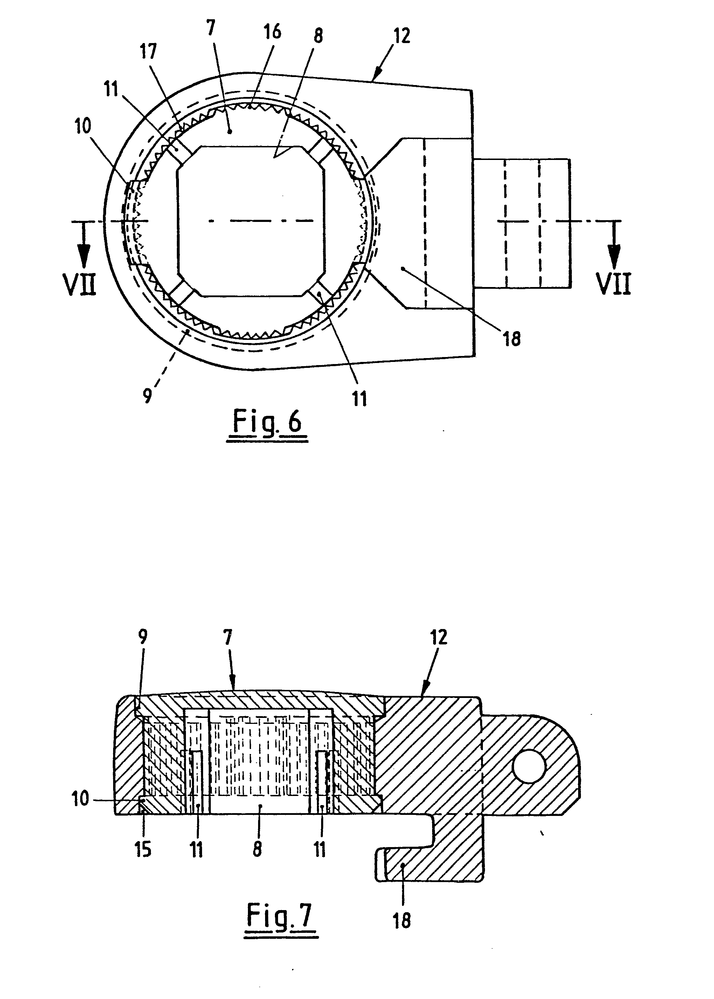

Figur 6 die Hülse mit eingesetzter Buchse in vergrößertem Maßstab in Ansicht und

Figur 7 einen Schnitt nach der Linie VII-VII der Figur 6.

Mit 1 ist der aus Kunststoff bestehende Brillenrahmen bezeichnet, dessen Brillenglasfassungen einen Schlitz S aufweisen. An der Rückseite des Brillenrahmens 1 sind zwei Seitenblenden 2 angelenkt. An dem Brillenrahmen 1 bzw. an den Brillenglasfassungen ist seitlich je ein vierkantiger, in Längsrichtung geteilter Zapfen 3 angeformt, dessen Teilungsebene in die Ebene des Schlitzes S der Brillenglasfassung übergeht. Mit 4 ist ein Ohrenbügel bezeichnet. Dieser ist mit seinem einen Ende in einem hülsenförmigen Körper 5 verschiebbar geführt. Der Mantel des Körpers 5 weist Rastlöcher 6 auf, in die der Ohrenbügel 4 mit Nocken einrastbar ist. Der Ohrenbügel 4 und der hülsenförmige Körper 5 bestehen ebenfalls aus Kunststoff. Mit 7 ist eine aus Kunststoff bestehende Buchse bezeichnet, die einen dem Zapfen 3 entsprechenden Innenvierkant 8 aufweist. Die Buchse 7 ist an dem Ende, mit dem sie dem Brillenrahmen 1 abgekehrt ist, geschlossen und weist hier einen kreisrunden Vorsprung 9 auf. Sie besitzt an ihrem anderen offenen Ende zwei nach auswärts gerichtete nasenartige Vorsprünge 10. Der Mantel der Buchse 7 weist in das offene Ende der Buchse 7 auslaufende Schlitze 11 auf. Mit 12 ist eine aus Kunststoff bestehende Hülse bezeichnet, die mit dem hülsenförmigen Körper 5 des Ohrenbügels 4 mittels eines Hohlnietes 13 gelenkig verbunden ist. Die Buchse 7 liegt mit ihren Vorsprüngen 9, 10 in Randausnehmungen 14, 15 der Hülse 12. Beim Einsetzen der Buchse 7 mit ihrem offenen Ende in die Hülse 12 wird durch die Schlitze 11 der Mantel der Buchse 7 um ein geringes Maß zusammengedrückt, so daß die Buchse 7 ungehindert durch die Vorsprünge 10 in die Hülse 12 eingedrückt werden kann. Der Mantel der Buchse 7 weist am äußeren Umfang Riffelungen 16 und die Hülse 12 am inneren Umfang Riffelungen 17 auf. Die Riffelungen 16, 17 bilden eine Rastsperre. Sie sind in Gruppen so aufgeteilt, daß zwischen den einzelnen Gruppen Freiräume vorhanden sind. Die Buchse 7 greift beim Einsetzen in die Hülse 12 mit ihren Riffelungen 16 in die Freiräume zwischen den zu Gruppen zusammengefaßten Riffelungen 17 der Hülse 12 ein. Durch eine Drehbewegung der Hülse 12 kommen die Riffelungen 16, 17 in Eingriff. Nach Montage der Buchse 7 wird die Hülse 12 mit dem Innenvierkant 8 der Buchse 7 auf den Zapfen 3 aufgeschoben. Um unbeabsichtigtes Lösen der Hülse 12 bzw. der Buchse 7 von dem Zapfen 3 zu verhüten, weist die Hülse 12 an der dem Brillenrahmen 1 zugekehrten Seite einen mit der Hülse 12 einstückigen, winkelförmigen Arm 18 auf, mit dem sie einen an dem Brillenrahmen 1 angeformten Vorsprung 19 untergreift. Zum Lösen des Ohrenbügels 4 von dem Zapfen 3 muß zuvor der Ohrenbügel 4 so weit um die Buchse 7 verschwenkt werden, bis der Arm 18 von dem Vorsprung 19 freigegeben ist.

## Patentansprüche

1. Brille, insbesondere Schutzbrille, mit geschlitzten Brillenglasfassungen und veränderbarer Inklination der Ohrenbügel, bei der ein jeder Ohrenbügel mit einer mit ihm gelenkig verbundenen Hülse auf einem an dem Brillenrahmen seitlich fest angeordneten, in Längsrichtung geteilten Zapfen gelagert ist, dessen Teilungsebene in die Schlitzebene der Brillenglasfassung übergeht, und bei der der Ohrenbügel in der zum Brillenrahmen eingestellten geneigten Lage mittels einer aus ineinandergreifenden Riffelungen bestehenden Rastsperre fixierbar ist, wobei die einen Riffelungen am inneren Umfang der Hülse des Ohrenbügels angeordnet sind, dadurch gekennzeichnet, daß

a) in die Hülse (12) des Ohrenbügels (4) eine Buchse (7) eingesetzt ist, bei der die mit den am inneren Umfang der Hülse (12) angeordneten Riffelungen (17) der Rastsperre in Wirkverbindung stehenden Riffelungen (16) außen am Mantel angeordnet sind,

b) die Buchse (7) gemeinsam mit der Hülse (12) auf den an dem Brillenrahmen (1) angeordneten Zapfen (3) drehfest aufgeschoben ist.

2. Brille nach Anspruch 1, dadurch gekennzeichnet, daß die Riffelungen (16, 17) der Rastsperre in Gruppen so aufgeteilt sind, daß zwischen den einzelnen Gruppen Freiräume vorhanden sind.

3. Brille nach Anspruch 1 oder 2, dadurch gekennzeichnet, daß

a) die Buchse (7) an ihrem einen Ende geschlossen ist und einen kreisrunden Vorsprung (9) besitzt,

b) die Buchse (7) an ihrem anderen Ende nach auswärts gerichtete, sich gegenüberliegende nasenartige Vorsprünge (10) besitzt,

c) die Buchse (7) mit ihren Vorsprüngen (9, 10) in Randausnehmungen (14, 15) der Hülse (12) liegt,

d) der Mantel der Buchse (7) in das offene Ende der Buchse (7) auslaufende Schlitze (11) aufweist.

4. Brille nach einem der vorherigen Ansprüche, dadurch gekennzeichnet, daß der Zapfen (3) vierkantig ist und die Buchse (7) einen entsprechenden Innenvierkant (8) besitzt.

## Claims

1. Spectacles, in particular safety goggles, with slotted rims for the lenses and an adjustable inclination of the side-pieces, in which each side-piece is mounted by means of a sleeve pivotably connected thereto on a pin which is fixedly arranged at the sides of the spectacle frame and divided in the longitudinal direction, the plane of division merging into the plane of the slots of the rims for the lenses, the side-piece being fixable in the adjusted inclined position relative to the spectacle frame by means of a stop notch consisting of intermeshing corrugations, the one corrugations being arranged at the inner circumference of the sleeve of the side-piece, characterised in that

a) a bush (7) is inserted into the sleeve (12) of the side-piece (4) in which the corrugations (16) cooperating with the corrugations (17) of the stop notch arranged at the inner circumference of the sleeve (12) are arranged at the outside of the casing,

b) the bush (7) together with the sleeve (12) is pushed onto the pin (3) arranged at the spectacle frame (1) in a manner secured against rotation.

2. Spectacles as claimed in claim 1, characterised in that the corrugations (16, 17) of the stop notch are divided into groups in such a manner that between the individual groups free spaces are provided.

3. Spectacles as claimed in claim 1 or 2, characterised in that

a) the bush (7) is closed at one of its ends and possesses a circular projection (9),

b) the bush (7) possesses nose-like projections (10) at its other end which are outwardly directed and arranged opposite to one another,

c) the bush (7) is placed with its projections (9, 10) in marginal recesses (14, 15) of the sleeve (12),

d) the casing of the bush (7) is provided with slots (11) running out into the open end of the bush (7).

4. Spectacles as claimed in one of the preceding claims, characterised in that the pin (3) is square and the bush (7) possesses a corresponding recessed square (8).

**Revendications**

1. Lunettes, en particulier lunettes protectrices, à montures fendues et inclinaison variable des branches, où chacune des branches est montée, au moyen d'un manchon flexiblement joint aux branches, sur un tenon divisé dans le sens de la longueur et fixé latéralement au châssis et dont le plan de division passe dans le plan de fente de la monture, et où la branche peut être fixée, dans la position inclinée réglée par rapport au châssis, au moyen d'un dispositif d'arrêt à crans constitué par des cannelures engrenantes dont les unes sont disposées au pourtour intérieur du manchon de la branche, lunettes caractérisées par ce que

a) dans le manchon (12) de la branche (4) est logée une douille (7) dont la chemise possède des cannelures extérieures (16) se trouvant en prise active avec les cannelures (17) disposées au pourtour intérieur du manchon (12) et constituant le dispositif d'arrêt à crans,

b) la douille (7) est engagée, en commun avec le manchon (12), sur le tenon (3) disposé au châssis (1), sans pouvoir pivoter.

2. Lunettes suivant revendication 1, caractérisées par ce que les cannelures (16, 17) du dispositif d'arrêt à crans sont réparties en groupes de manière à laisser des espaces libres entre les différents groupes.

3. Lunettes suivant revendication 1 ou 2, caractérisées par ce que

a) la douille (7) est fermée à l'une de ses extrémités et qu'elle possède une saillie circulaire (9),

b) la douille (7) possède à son autre extrémité des saillies (10) opposées formant talon et dirigées vers l'extérieur,

c) la douille (7) avec ses saillies (9, 10) loge dans des évidements (14, 15) dans les bords du manchon (12),

d) la chemise de la douille (7) présente des fentes (11) sortant dans l'extrémité ouverte de la douille (7).

4. Lunettes suivant une des revendications précédentes, caractérisées par ce que le tenon (3) est carré et que la douille (7) possède un carré intérieur (8) correspondant.

**Fig.1**

0 076 499

Fig. 2

Fig. 3

Fig. 4

Fig. 5

0 076 499

Fig. 6

Fig. 7